# FASCICULE DE BREVET EUROPEEN

(11) **EP 0 682 920 B1**
(45) Date de publication et mention de la délivrance du brevet: **03.05.2000**
(21) Numéro de dépôt: 95440006.5
(22) Date de dépôt: 14.02.1995
(51) Int. Cl.: A61B 19/02

(54) **Dispositif de conditionnement d'un objet en métal à mémoire de forme**
Verpackung für einen aus Formgedächtnismetall bestehenden Gegenstandes
Packaging for object made of shape memory metal

(30) Priorité: 15.02.1994 FR 9401843
(43) Date de publication de la demande: 22.11.1995
(73) Titulaire: Depuy France, 69100 Villeurbanne (FR); Barouk, Louis Samuel, F-33370 Yvrac (FR)
(72) Inventeur: Augagneur Christian, F-69007 LYON (FR)
(74) Mandataire: Polus, Camille

(56) Documents cités:
- EP-A- 0 145 166
- EP-A- 0 397 607
- EP-A- 0 488 906
- WO-A-91/12771
- FR-A- 2 537 439
- SU-A- 1 230 593
- US-A- 3 696 920
- US-A- 5 082 112

## Description

La présente invention a pour objet un dispositif de conditionnement en position d'utilisation d'un objet en métal à mémoire de forme, notamment une agrafe de chirurgie orthopédique, réalisé selon le préambule de la revendication 1. Un tel dispositif est connu par le document US-A-3696920.

On utilise actuellement de plus en plus fréquemment des objets en métal à mémoire de forme qui présentent la particularité de prendre des formes différentes selon la température à laquelle ils sont exposés.

Par exemple dans le domaine de la chirurgie orthopédique on utilise des agrafes faites d'un tel métal dans le but de réaliser plus aisément la consolidation et le rapprochement de deux parties osseuses.

Une telle agrafe comporte généralement, mais non limitativement, deux branches qui, lorsque ladite agrafe est maintenue à basse température, peuvent être écartées l'une de l'autre et positionnées parallèlement l'une à l'autre, le métal utilisé étant alors relativement malléable, tandis que le réchauffement de l'agrafe à la température ambiante, ou à la température du corps, provoque le rapprochement spontané des extrémités, qui restent néanmoins rectilignes, et ce de manière irréversible à ladite température.

En pratique, les agrafes sont conservées stériles dans un endroit très froid, du type congélateur, d'où elles sont extraites au moment de leur utilisation.

L'inconvénient est que le congélateur doit être à proximité du lieu d'opération, et que l'agrafe doit être utilisée immédiatement après sa sortie dudit congélateur.

Or, au moment de la pose d'une agrafe, le chirurgien peut être confronté à une difficulté l'empêchant d'utiliser immédiatement ladite agrafe, qui se réchauffe et devient alors inutilisable, ce qui nécessite l'emploi d'une autre agrafe.

A cet égard, le dispositif du document US-A-3 696 920 ne fournit aucune solution à ce problème, car il n'est pourvu d'aucun moyen de conservation provisoire du froid, est n'est d'ailleurs nullement destiné à recevoir des agrafes à mémoire de forme.

La présente invention vise à remédier à cet inconvénient en proposant un dispositif de conditionnement en position d'utilisation d'un objet en métal à mémoire de forme, notamment une agrafe de chirurgie orthopédique, permettant de conserver celui-ci prêt à l'emploi pendant un certain temps après qu'il ait été sorti de son lieu de conservation.

Le dispositif de conditionnement selon la présente invention se caractérise essentiellement par les caractéristiques de la partie caractérisante de la revendication 1.

L'objet à conserver, après mise en place dans le support, est placé dans la boîte, qui est maintenue à basse température jusqu'à son utilisation.

La mise en oeuvre d'un tel support adapté à la forme de l'objet prêt à l'emploi permet de maintenir ce dernier dans ladite forme pendant un certain laps de temps après sa sortie de son lieu de conservation, en raison notamment du rôle thermique joué par le support.

Ledit objet peut ainsi être maintenu dans sa forme d'utilisation pendant une durée de l'ordre de 10 à 20 minutes, fonction d'une part de la température de conservation de l'objet et d'autre part de la température ambiante lors de sa mise en place.

L'empreinte femelle d'un support selon l'invention appliqué au conditionnement d'une agrafe de chirurgie orthopédique à deux branches peut être constituée de deux canaux sensiblement parallèles dans chacun desquels est introduite une branche de l'agrafe, permettant au chirurgien de disposer à portée de main d'une agrafe prête à l'emploi.

Le support peut d'autre part comporter, dans sa partie opposée à celle où est pratiquée l'empreinte femelle, des moulures facilitant sa préhension.

Selon un mode de réalisation particulier du dispositif selon l'invention, la boîte obturable hermétiquement comporte au moins une paroi réalisée dans un matériau à fort pouvoir de conservation du froid.

Ladite paroi en matériau à fort pouvoir de conservation du froid de la boîte est de préférence le fond de celle-ci, réalisable par mise en place d'un tel matériau, dans lequel peut être pratiquée une empreinte creuse dont la forme permet une introduction totale ou partielle du support de l'objet.

Un dispositif de ce type permet de rallonger sensiblement la durée de conservation de l'objet, qui peut en ce cas aller jusqu'à plusieurs heures.

Il est à noter que selon un autre mode de réalisation, le support et l'objet peuvent être enfermés dans une boîte de type classique elle-même introduite dans une seconde boîte dont au moins une paroi est réalisée dans un matériau à fort pouvoir de conservation du froid.

A titre de matériau à fort pouvoir de conservation du froid utilisable dans ce type de conditionnement on peut citer les liquides de densité supérieure ou égale à la densité de l'eau ; les produits liquides ou solides dont le point d'ébullition ou de sublimation est très bas, comme l'azote liquide ou la neige carbonique ; les liquides volatils comme l'éther ou les fréons ; les produits solides dont la mise en solution consomme une grande quantité d'énergie, comme le nitrate de soude en solution aqueuse.

Dans le cas où l'on met en oeuvre un produit liquide, celui-ci est enfermé dans une enveloppe étanche qui peut être placée sur le fond de la boîte, dont elle épouse de préférence les contours.

Les avantages et les caractéristiques de la présente invention ressortiront plus clairement de la description qui suit et qui se rapporte au dessin annexé, lequel en représente deux modes de réalisation non limitatifs.

Dans le dessin annexé :
- la figure 1 représente une vue en perspective avec arraché partiel d'un dispositif selon l'invention appliqué au conditionnement d'une agrafe chirurgicale.
- la figure 2 représente une vue en perspective du support d'agrafe de la figure 1.
- la figure 3 représente une vue en coupe longitudinale d'une variante du support d'agrafe.
- la figure 4 représente une vue en coupe selon l'axe 4-4 de la figure 3
- la figure 5 représente une vue en coupe selon l'axe 5-5 de la figure 3.

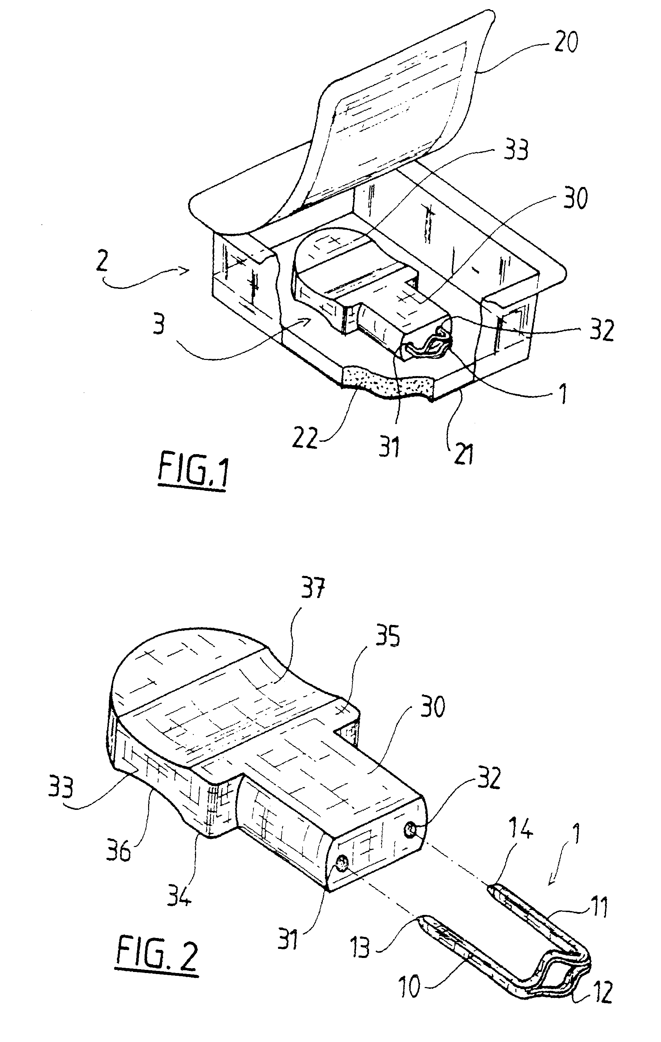

Si on se réfère à la figure 1 on peut voir qu'un dispositif de conditionnement selon l'invention d'une agrafe 1 en métal à mémoire de forme comprend une boîte 2 et un support 3.

La boîte 2 est de préférence en matière plastique transparente afin de permettre de voir son contenu, et est close de manière étanche au moyen d'un couvercle 20, par exemple du type film de papier serti à chaud, l'intérieur de la boîte 2 et son contenu étant stérilisés, par exemple par rayonnement gamma.

Le fond 21 de la boîte 2 est recouvert d'une couche 22 d'une certaine épaisseur d'un matériau à fort pouvoir de conservation du froid, qui peut éventuellement comporter une empreinte creuse, non représentée, permettant l'insertion totale ou partielle du support 3.

Si on se réfère également à la figure 2 on peut voir sur cette figure une agrafe 1 de forme générale en U à deux branches 10 et 11 reliées par une partie transversale 12, qui telles que représentées sont parallèles, position qui ne peut être prise qu'à basse température. En effet en cas de réchauffement de l'agrafe 1 les branches 10 et 11 s'inclinent, les angles qu'elles forment avec la partie transversale 12 se ferment, en sorte que les extrémités libres respectivement 13 et 14 des branches 10 et 11 se rapprochent.

Le support 3 de l'agrafe 1 comporte une partie antérieure 30 dont l'extrémité est percée de deux canaux parallèles 31 et 32 destinés à abriter chacune des branches, respectivement 10 et 11, de l'agrafe 1, lorsque celles-ci sont parallèles et donc prêtes à l'emploi.

La partie postérieure 33 du support 3 a une forme permettant son maintien lors de l'extraction de l'agrafe 1, les faces inférieure 34 et supérieure 35 comportant chacune à cet effet une large gorge transversale, respectivement 36 et 37.

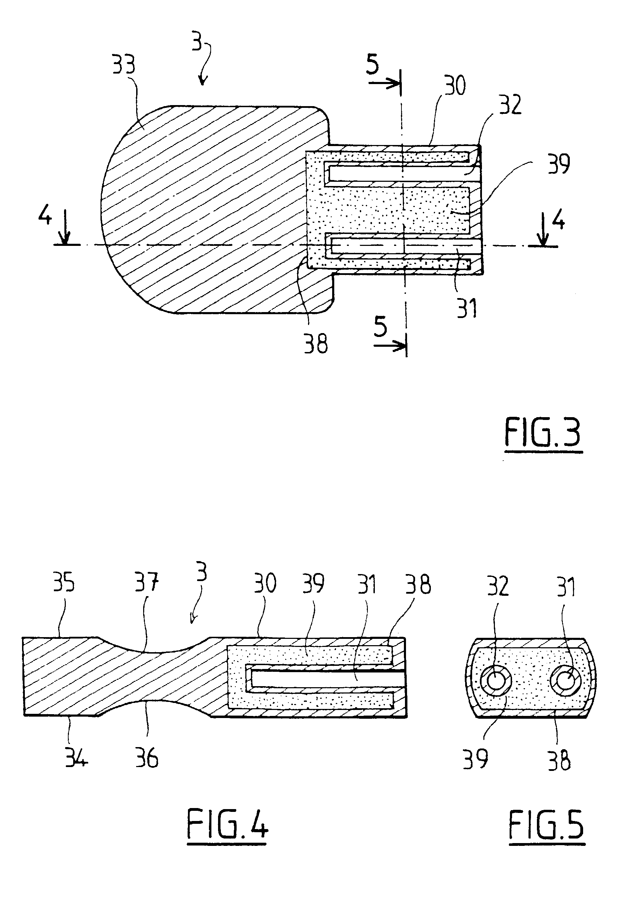

Si on se réfère maintenant aux figures 3, 4 et 5 on peut voir que le support 3 peut comporter intérieurement une cavité fermée 38 enveloppant les canaux 31 et 32, emplie d'un matériau 39 à fort pouvoir de conservation du froid, du même type que celui dont est constituée la couche 22 de la boîte 2.

Il est à noter que pour un support d'agrafe 1 constitué d'une entretoise, la variante décrite ci-dessus est également réalisable, dans ce cas,non représenté, la cavité fermée 38 enveloppe les gorges 42 et 43.

## Revendications

1. Dispositif de conditionnement en position d'utilisation d'un objet en métal à mémoire de forme, notamment une agrafe de chirurgie orthopédique, comportant d'une part un support (3, 4) comportant une partie (30, 4) dans laquelle est pratiquée une empreinte femelle (31, 32 ; 42, 43) correspondant à la forme dudit objet (1) lorsqu'il est prêt à l'emploi, et d'autre part une boîte (2) obturable hermétiquement destinée à renfermer ledit objet (1) placé dans ledit support (3, 4), caractérisé en ce que le support (3, 4) comporte intérieurement une cavité (38) enveloppant l'empreinte femelle (31, 32 ; 42, 43), emplie d'un matériau (39) à fort pouvoir de conservation du froid.

2. Dispositif selon le revendication 1 caractérisé en ce que le support (3) comporte, dans sa partie (33) opposée à celle, comprenant l'empreinte femelle (31, 32), des moulures (36, 37) permettant sa préhension.

3. Dispositif selon l'une des revendications précédentes, caractérisé en ce que la boîte (2) comprend au moins une paroi réalisée en un matériau à fort pouvoir de conservation du froid.

4. Dispositif selon la revendication 3 caractérisé en ce que la paroi en matériau à fort pouvoir de conservation du froid de la boîte (2) est le fond (21) de celle-ci, sur lequel est mise en place une couche (22) d'un tel matériau.

5. Dispositif selon la revendication 3 ou 4 caractérisé en ce que la paroi en matériau à fort pouvoir de conservation du froid comporte une empreinte creuse dont la forme permet une introduction totale ou partielle du support (3, 4).

6. Dispositif selon l'une quelconque des revendications 3 à 5, caractérisé en ce que le matériau à fort pouvoir de conservation du froid est choisi dans le groupe formé par les liquides de densité supérieure ou égale à la densité de l'eau ; les produits liquides ou solides dont le point d'ébullition ou de sublimation est très bas, comme l'azote liquide ou la carboglace ; les liquides volatils comme l'éther ou les fréons ; les produits solides dont la mise en solution consomme une grande quantité d'énergie, comme le nitrate de soude en solution aqueuse.

7. Dispositif selon l'une quelconque des revendications précédentes, caractérisé en ce que le support (3, 4) est réalisé en matière plastique moulée.

## Patentansprüche

1. Vorrichtung zum Bereitstellen eines Metaligegenstands mit Formgedächtnis, insbesondere einer orthopädischen chirurgischen Klammer in Gebrauchsposition, aufweisend einwärts einen Träger (3, 4) mit einem Abschnitt (30, 40), in welchem eine Eindrückung (31, 32; 42, 43) entsprechend der Form des gebrauchsfertigen Gegenstands (1) und andererseits eine hermetisch verschließbare Kapsel vorgesehen sind, die dazu bestimmt ist, den in dem Träger (3, 4) angeordneten Gegenstand (1) einzuschließen,
dadurch gekennzeichnet, daß der Träger (3, 4) im Innern einen Hohlraum (38) aufweist, welcher die Eindrückung (31, 32; 42, 43) umschließt, der mit einem Material (39) mit hohem Kältespeichervermögen versehen ist.

2. Vorrichtung nach Anspruch 1, dadurch gekennzeichnet, daß der Träger in seinem die Eindrückung (31, 32) enthaltenden, ihm gegenüberliegenden Teil (33) sein £rgreifen ermöglichende Stege (36, 37) umfaßt.

3. Vorrichtung nach einem der vorangehenden Ansprüche, dadurch gekennzeichnet, daß die Kapsel () zumindest eine Wand umfaßt, die aus einem Material mit hohem Kältespeichervermögen gebildet ist.

4. Vorrichtung nach Anspruch 3, dadurch gekennzeichnet, daß die Wand aus einem Material mit hohem Kältespeichervermögen der Kapsel (2) den Boden derselben bildet, auf welchem eine Schicht (22) aus einem derartigen Material in Stellung gebracht ist.

5. Vorrichtung nach Anspruch 3 oder 4, dadurch gekennzeichnet, daß die Wand aus einem Material mit hohem Kältespeichervermögen eine Eintiefung umfaßt, deren Form die vollständige oder teilweise Einführung des Trägers (3, 4) erlaubt.

6. Vorrichtung nach einem der Ansprüche 3 bis 5, dadurch gekennzeichnet, daß das Material mit hohem Kältespeichervermögen aus einer Gruppe gewählt ist, die gebildet ist durch Flüssigkeiten einer Dichte gleich der Dichte von Wasser oder höher als diese, durch flüssige oder feste Stoffe, deren Siede- oder Sublimationspunkt sehr niedrig liegt, wie etwa flüssiger Stickstoff oder Trokkeneis, durch flüchtige Flüssigkeiten, wie etwa Ether oder Freone, durch Feststoffe, deren Auflösung eine große Energiemenge erfordert, wie Natronnitrat in wäßriger Lösung.

7. Vorrichtung nach einem der vorangehenden Ansprüche, dadurch gekennzeichnet, daß der Träger (3, 4) aus Formpreßstoff gebildet ist.

## Claims

1. Device for packaging a metal object with a shape memory, notably a clip for orthopaedic surgery, in the position of use, comprising on the one hand a support (3, 4) having a part (30-, 4) in which is formed a female impression (31, 32; 42, 43) corresponding to the shape of said object (1) when it is ready for use, and on the other hand a hermetically sealable box (2) intended to contain said object (1) placed in said support (3, 4), characterised in that the support (3, 4) comprises on the inside a cavity (38) surrounding the female impression (31, 32; 42, 43), filled with a material (39) with a high cold-retaining power.

2. Device according to claim 1, characterised in that the support (3) comprises, in its part (33) opposite the part comprising the female impression (31, 32), moulded areas (36, 37) enabling it to be gripped.

3. Device according to one of the preceding claims, characterised in that the box (2) comprises at least one wall made of a material with a high cold-retaining power.

4. Device according to claim 3, characterised in that the wall of the box (2) made of a material with a high cold-retaining power is the bottom (21) of the box (2), on which is positioned a layer (22) of such a material.

5. Device according to claim 3 or 4, characterised in that the wall made of a material with a high cold-retaining power has a hollow impression the shape of which enables the support (3, 4) to be placed wholly or partly therein.

6. Device according to any one of claims 3 to 5, characterised in that the material with a high cold-retaining power is selected from among the liquids with a density greater than or equal to the density of water; the liquid or solid products having a very low boiling or sublimation point, such as liquid nitrogen or dry ice; the volatile liquids such as ether or the freons; the solid products whose dissolution consumes large amounts of energy, such as sodium nitrate in aqueous solution.

7. Device according to any one of the preceding claims, characterised in that the support (3, 4) is made of moulded plastics.
